# EUROPEAN PATENT SPECIFICATION

(11) **EP 1 135 031 B1**
(45) Date of publication and mention of the grant of the patent: **13.12.2006**
(21) Application number: 99959440.1
(22) Date of filing: 30.11.1999
(51) Int. Cl.: A23K 1/18, C12Q 1/00, G01N 33/02

(54) **METHOD FOR TESTING A FODDER COMPONENT FOR THE FEEDING OF RUMINANTS**
TESTVERFAHREN FUER FUTTERMITTELKOMPONENTE FUER DAS FUETTERN VON WIEDERKAEUERN
PROCEDE POUR TESTER UN PRODUIT DE FOURRAGE UTILISE POUR NOURRIR DES RUMINANTS

(30) Priority: 01.12.1998 FI 982598
(43) Date of publication of application: 26.09.2001
(73) Proprietor: SUOMEN REHU OY, 00500 Helsinki (FI)
(72) Inventor: VIRKKI, Markku, FIN-02320 Espoo (FI); NURMINEN, Päivi, FIN-02430 Masala (FI); APAJALAHTI, Juha, FIN-00670 Helsinki (FI)
(74) Representative: Partio, Erja
(86) International application number: PCT/FI1999/000993
(87) International publication number: WO 2000/032059

(56) References cited:
- WO-A1-97/06434
- M.C.N. JAYASURIYA ET AL.: 'The use an Artificial Rumen to Assess Low Quality Fibrous Feeds' BIOLOGICAL WASTES, vol. 20, 1987, (GREAT BRITAIN), pages 241 - 250, XP002947827
- J.L. HORNER ET AL.: 'Effects of Whole Cottonseed, Niacin and Niacinamide on In Vitro Rumen Ferentation and on lactating Holstein Cows' JOURNAL OF DAIRY SCIENCE, vol. 71, no. 12, 1988, (TEXAS), pages 3334 - 3344, XP002947828
- H.R. MANSFIELD ET AL.: 'Effects of Beet pulp and Animal By-Products on Milk Yield and In Vitro Fermentation by Rumen Microorganisms' JOURNAL OF DAIRY SCIENCE, vol. 77, no. 1, 1994, (MINNESOTA), pages 205 - 216, XP002947829
- Y. DONG ET AL.: 'Lipid-induced depression of methane production and digestibility in the artificial rumen system (RUSITEC)' CANDADINA JOURNAL OF ANIMAL SCIENCE, vol. 77, no. 2, 1997, (CANADA), pages 269 - 278, XP002948933

## Description

The present invention relates to a method as defined in the preamble of claim 1 for testing a fodder component for the feeding of ruminants.

For ruminants, the effect of a diet is generally tested with large numbers of animals. In research plants, the animals are traditionally sorted into groups as uniform as possible in respect of age, yield, living weight, etc. In the tests, large group sizes have to be used to compensate for variations so that the differences between groups can be brought out statistically. Likewise, for field experiments carried out on farms, the animals are sorted on certain grounds into groups as uniform as possible. Because of the variations between farms, the size of the groups must be very large, e.g. at least 800 individuals according to the Swedish Lantmännen, to achieve statistical reliability.

Prior-art methods for the planning of the feeding of animals are based on the animals' statistical need for nutrients. The feeding of animals is based on satisfying their statistical needs for energy, proteins, minerals, vitamins, etc. In the planning of feeding, the percentages of nutrients in the fodder are generally determined for the various separate fodder components using tables.

The publication Biological Wastes, vol. 20, pages 241-250 discloses the use of artificial rumen to asses low quality fibrous feeds.

The publication Journal of Dairy Science, vol. 71, no. 12, 1988, page 3334-3344 discloses the experiments about the effects of whole cottonseeds, niacins and niacinamines on in vitro rumen fermentation and on lactating Holstein cows.

The publication Journal of Dairy Science, vol. 77, no.1, 1994, pages 205-216 discloses effects of beef pulp and animal by-products on milk yield and in vito fermentation by rumen microorganisms.

The publication Canadian Journal of animal science, vol. 77, no. 2, 1997, pages 269-278 discloses lipid-induced depression of methane production and digestibility in the artificial rumen system (RUSITEC).

The publication WO 9706434 discloses feed digestibility measurement in ruminants.

Previously known research plant and field testing methods are not applicable for the determination of the effects of a diet in the case of a small group of animals. A problem with prior-art testing methods is a large divergence within groups, which makes it necessary to increase the group size in order to achieve results that are statistically significant. Increasing the group size naturally increases the costs of the tests and leads to difficulties in the organization of the tests and the treatment of the numeral material.

A further problem is that determining the nutritive values of new fodder components and therefore the development of diets by current methods is an expensive and slow process. For new fodder components, the results are always uncertain without extensive field experiments.

The object of the invention is to eliminate the problems referred to above and to disclose a new and usable method for the testing of a fodder component, especially in respect of its productional effect. A further object of the invention is to disclose a method which is more accurate as well as easier and cheaper to implement than previously known methods.

Another object of the invention is to disclose a method which can be used to determine environmental effects of fodders, especially as regards the final emissions.

The method of the invention is characterized by what is presented in the claims.

The invention is based on testing a fodder component for the feeding of ruminants using ruminal fermentation simulation known in itself. In ruminal fermentation simulation, one or more fodder components are tested to determine their properties, and it is performed in a substantially artificial rumen so that the fodder component is decomposed by the action of microbes into fermentation products utilizable in the animal's metabolism and providing the nutritive substances needed by the animal. In the method of the invention, fermentation products substantially proportional to the animal's yield, e.g. the quantity and/or quality of yield, are measured, and the fermentation products are compared with corresponding results achieved using known fodder components and/or diets whose productional effect is known. Based on the comparison, the effect of the fodder component on the animal's yield is determined. The yield may consist of any product obtained from the animal, e.g. milk.

The results of ruminal fermentation simulation preferably correspond to the decomposition results and/or products produced in the conditions prevailing in the animal's rumen, because the conditions and microbes in the artificial rumen substantially correspond to the conditions and microbes in the animal's rumen.

In an embodiment of the invention, the effect of the fodder component on the yield is determined on the basis of one or more cycles of ruminal fermentation simulation.

In an embodiment of the invention, the method comprises measuring the microbial mass produced. In an embodiment, the number of microbes per unit of weight and/or volume is measured.

In an embodiment of the invention, the fixation of nitrogen in the microbial mass is determined.

In an embodiment of the method of the invention, the total gas production, total acids, acetic acid, propionic acid, butyric acid and/or total profile of the products are measured. The acids may include e.g. various organic acids, such as volatile fatty acids. Of course, the method may comprise the measurement of any products produced in ruminal fermentation.

In an embodiment of the invention, the fermentation products can be measured qualitatively and/or quantitatively substantially during and/or after fermentation.

In an embodiment of the invention, a fodder component tested by ruminal fermentation simulation is tested via a field experiment. In the field experiment, the animals are divided into a control group and a test group, which are fed with a substantially similar basic diet, so that e.g. when there is a change in the quality of ensilage, the changes will occur simultaneously in both groups. The fodder component to be tested is included in the diet of the animals in the test group during a certain period of time. The yield of the animals in the control and test groups as well as the effect of the fodder component on the yield are measured by comparing the yields of the two groups with each other. The animals can be divided into groups in a random manner e.g. so that every second animal belongs to the control group and every second animal to the test group.

In an embodiment of the invention, the effect of the fodder component on the yield is determined via a field experiment by comparing the measured yield of the test group and control group with a reference level, which is defined by measuring the yield of each animal and/or group for a given earlier or later period of time during which each animal is fed a substantially uniform diet. The reference level can be defined e.g. by measuring the yield of the animal during a period e.g. of one year. This period may naturally be of any length.

In an embodiment of the invention, the effect of the fodder component on the yield is determined via a field experiment before and/or after calving. Via a field experiment, statistically significant differences regarding the fodder component and the yield can be determined by using statistical methods known in themselves.

In an embodiment of the invention, the milk production of cows is tested via a field experiment and a test milking is performed at given intervals of time, e.g. at intervals of one month, to measure the yield. Naturally, test milking intervals of any length may be used. Similarly, any number of test milkings may be performed.

The method presented in claims 9 - 14 can naturally also be applied without ruminal fermentation simulation or in conjunction with some other simulation method.

By using the method of the invention, the effect of new fodder components and/or a new diet on the yield in respect of quantity and time can be easily and quickly established. The method can be applied in the planning of diets e.g. for cattle in accordance with the fodder resources available on the market depending on the availability, price, quality etc. of the fodder component.

A further advantage of the invention is that the actual, effective nutritive values of the fodder component can be determined with regard to its productional effect via ruminal fermentation simulation.

Moreover, the invention allows easy investigation and comparison of fodder samples received from different parts of the world with regard to the productional effect of the samples.

A further advantage of the invention is that it makes it possible to determine the fixation of nitrogen in the microbial mass, allowing the animal's diet to be so defined that it does not contain any large amounts of so-called surplus nitrogen. Uncombined nitrogen may have an effect on the environment and on the animal's stress and fecundity because nitrogen not fixed in microbes escapes from the rumen in the form of ammonia and is secreted mainly in urine in the form of urea. The conversion from ammonia to urea requires energy at the tissue level and extra ammonia at the tissue level may reduce fecundity.

The invention additionally has the advantage that, via field experimenting with only a small amount of animal material, it is possible to reveal statistically significant differences regarding diet and yield. At the same time, reliable material is produced which can be used in support of marketing efforts e.g. in the marketing of different fodder component products.

In the following, the invention will be described by the aid of detailed examples of its embodiments.

### Example 1; ruminal fermentation simulation

In an artificial rumen made e.g. of glass, an inoculum is used which comprises ruminal liquid taken from a cow and small fibers. The bacterial strains on the surface of the fibers are different from those in free liquid. For ruminal fermentation simulation, it is preferable to use the entire microbial community because some of the bacteria need the metabolic products secreted by other bacteria.

The inoculum is treated in a substantially oxygen-free space to ensure that the ruminal microbes remain as vital as possible. The temperature of the inoculum is kept near the ruminal temperature during the test.

In ruminal fermentation simulation, a phosphate or carbonate buffer is introduced into the rumen to prevent large pH variations due to intensive acidification in the rumen; a pH of 6,0 - 7,0, preferably about 6.8 is maintained. The concentration of the buffer solution used is changed when necessary, because e.g. a fodder mixture containing almost exclusively pure corn produces such intensive acidification that buffering has to be increased.

The amount of fodder used in the simulation bears a certain relation to the amount of buffer and inoculum, said relation mainly corresponding to the real rumen; for instance, a total volume of 40 ml contains 1 g of fodder and 7 ml of inoculum. Any ensilage contained in the fodder mixture is chopped. The fodder, inoculum and/or buffer solution are combined e.g. in an impermeable bottle under a protective gas, e.g. CO2. The rumen simulation is started as soon as possible after the inoculum has been taken, e.g. within 1 - 3 hours. The ruminal fermentation simulation, i.e. incubation of ruminal cultures, is performed at a steady temperature, e.g. 38°C, and its duration is about 12 h.

During the simulation, the progress of the fermentation is monitored by measuring the metabolic products of microbes, such as gases, organic acids, microbial mass and/or number of microbes.

The bacteria in the rumen produce organic acids, which the ruminant utilizes in its metabolism as an energy source; of the energy needed by the animal, 70 - 80 % can be satisfied by volatile fatty acids. The amount and quality of the acids produced are important factors determining the nutritive value of the fodder. For example, propionic acid is converted in the cow's liver into sugar and it constitutes a preliminary form of lactose and milk, which means that propionic acid improves the yield of the animal. The production of organic acids is monitored via gas chromatography during the fermentation.

For the ruminant, the production of organic acids is as important as the production of microbial mass. It forms a considerable proportion of the protein supply of the animal; of the protein supply needed by the animal, e.g. 70 - 100 % can be satisfied by the microbial mass. Microbial cells degrade in the alimentary canal after the rumen, releasing proteins and aminoacids so that the animal can utilize them. The production of microbial mass is measured in simulation tests by computing the number of microbes per unit of weight or volume during and/or after the test.

To determine the number of microbes, microbial cells are separated from the fiber fraction by differential centrifugation, in which 40 g of sample is mixed with 60 ml of a buffer which preferably has a pH of 4.8 and which consists of 0.2 M sodium acetate and 0.1 % TWEEN 80. The mixture is agitated for 10 min at room temperature and centrifuged for 15 min at a speed of 200 x g to pelletize undigested fodder, with the result that the microbial cells remain in the supernatant. The washing operation is repeated e.g. 3 times, 98 % of the microbial cells being thus reclaimed. The supernatants are combined and centrifuged for 15 min at a speed of 30000 x g to pelletize the microbial cells. After this, the microbes are stored in a buffer which preferably has a pH of 4.8 and which consists of 0.2 M sodium acetate and 4 % formaldehyde. The microbial cells are colored by adding a fluorescent dye to the solution, e.g. in an amount of 5 µl of DAPI solution (4,6-diamidino-2-phenyl-indol; 5 mg/ml) and incubating it at room temperature for 5 min. The solution, 1 ml, is filtered through a polycarbonate filter dyed black, e.g. Nuclepore^{®}, whereupon the filter is washed with 10 ml of water. The microbes thus rendered fluorescent are counted from the filter using e.g. a fluorescence microscope known in itself and an automatic counting program. From each simulation culture, e.g. 7 parallel counts are determined, and from each filter at least 10 fields are included in the computation.

Furthermore, rumen simulation may generate undesirable metabolic products. For instance, methanogenic bacteria in the rumen produce methane, which in the ruminal conditions is an inert gas without an energy value. For the environment, methane is an undesirable gas and the aim is to reduce its production. Methane can be analyzed via gas chromatography.

Different bacterial stocks produce above-mentioned metabolic products in different proportions depending on the fodder, so it is generally preferable to determine an overall profile of the products.

### Example 2; field experiment

A field experiment was carried out to study the effect of a product called Ca Balans on milk production after calving. In the experiment, the energy-corrected milk quantity, i.e. ECM, of the animals was measured.

The animals were divided at random into a control group of 10 animals and a test group of 14 animals. The yield of the animals for the previous year, i.e. the reference level, had been determined.

The animals in the test group were fed the Ca Balans product for about 3 weeks before calving and the diet was ended on the day of calving. The yield for the milking period which began after the calving was measured and compared with the reference level. The average test milking results of the control and test groups for a 3-month period are presented in Table 1; the energy-corrected milk quantity is given in kilograms (kg)/animal per day.

The results indicate that the yield of the animals increased during the milking period when 3 months had elapsed after the calving; the increase in the yield as compared with the previous year was 2.1 kg. At the same time, the yield of the control group increased by 0.2 kg as compared with the previous year. Thus, the net yield increase was 1.9 kg/day, in total 175 kg/3 months.

**Table 1**

| | Control group (10) | | | Test group (14) | | |
|---|---|---|---|---|---|---|
| | 1 month after calving | 2 months after calving | 3 months after calving | 1 month after calving | 2 months after calving | 3 months after calving |
| Reference year, ECM | 30,8 | 29,5 | 29,6 | 33,2 | 33,9 | 32,1 |
| Current milking period, ECM | 29,3 | 31, 3 | 29 , 9 | 32 , 4 | 35 , 7 | 37,4 |

### Example 3; rumen simulation and field experiment

The effect of a new fodder component on the yield, in this case milk production, was investigated via rumen simulation and field testing.

Betaine was separated from different vinasses, such as citric acid vinasse and yeast vinasses. Vinasse with a betaine content is formed when beet molasses are used as a substrate for micro-organisms producing citric acid or yeast mass. A small amount of separation molasses was mixed in vinasses from which betaine had been separated and the fodder component obtained was tested via ruminal fermentation simulation (Example 1) and a field experiment.

In the ruminal fermentation simulation, the fodder component proved to have properties at least as good as those of the pea when the volatile fatty acids VFA and efficiency of microbial synthesis were measured. In addition, the fodder component significantly reduced methane production in the rumen.

In the field testing, 2 kg of corn used in the feeding of the control group was replaced with the fodder component (2 kg). The fodder mixture used to feed the animals also contained other conventional fodder components. The fodder component under testing was used in the feeding of 107 highly productive animals (GR 3 & 4) for 1 month (August). The diet of the animals in the test group during the preceding and the following month was substantially similar to the diet of the control group and it did not include the fodder component to be tested. The control group in this test consisted of 58 less productive animals (GR 1 & 2). The milk yield, i.e. energy-corrected milk quantity ECM produced by the animals in these test and control groups was measured during a 3-month test period. Table 2 presents the average yield (kg) per day of both groups during the 3-month test period.

**Table 2**

| | Test group (107) | | | Control group (58) | | |
|---|---|---|---|---|---|---|
| | July | August | September | July | August | September |
| ECM | 32.4 | 32.1 | 29.6 | 20.6 | 18.4 | 16.2 |

The results indicate that removing the fodder component from the diet of the animals in the test group resulted in a loss of 1.1 kg in the yield. Simultaneous yield loss in the control group was 0.1 kg, so the net benefit from the fodder component was 1.2 kg. The fodder component (1 kg) tested thus corresponded to 1 kg of corn and a 0.6 kg increase in milk yield.

### Example 4; comparison of diets via rumen simulation

The diets used on two milk farms were compared via ruminal fermentation simulation experiments. Table 3 presents the daily animal-specific diet used on each milk farm (fodder components in terms of fresh weight (kg)).

**Table 3**

| | Farm 1 | Farm 2 |
|---|---|---|
| Ensilage | 28.9 | 21.4 |
| Corn | 0 | 7.9 |
| ELIT 2000 | 6.4 | 0 |
| ELIT 11F | 0 | 4.6 |
| AVANT 95 | 5.6 | 0 |
| BETFOR | 2 | 0 |
| Soy | 0 | 2 |
| Total fresh weight | 42.9 | 35.9 |

Fodder mixtures consistent with the diets were tested via ruminal fermentation simulation. In the experiment, 10 parallel simulation cultures for both diets were tested. Table 4 presents the average quantities of the fermentation products produced in the simulation tests and the divergence of the results as well as statistically significant differences between the diets.

The results indicate that different diets produce significantly diverging simulation results regarding gas production, total acids and number of microbes. The diet used on farm 1 favors the production of organic acids in ruminal fermentation. The diet used on farm 2 increases microbial synthesis in the rumen. The results indicate that the divergence regarding the measured fermentation products is so small that the differences between the diets are significantly revealed statistically.

**Table 4**

| | Amount of Fermentation products | | Statistical differences |
|---|---|---|---|
| | Farm 1 | Farm 2 | Farm 1 vs Farm 2 |
| Gas production | | | |
| 12 h, (ml) | 185 | 170 | p < 0.0001 |
| S.E. | 1.6 | 1.2 | |
| Delay | 0.33 | 1.84 | |
| Total acids (mM) | 109 | 97 | p = 0.008 |
| S.E. | 3.21 | 1.15 | |
| Acetic acid, % | 48 | 48 | p = 0.870 |
| S.E. | 0.9 | 0.4 | |
| Propionic acid, % | 34 | 35 | p = 0.280 |
| S.E. | 0.6 | 0.2 | |
| Butyric acid, % | 14 | 12 | p = 0.008 |
| S.E. | 0.4 | 0.1 | |
| Number of microbes, | 3.3 | 4.1 | p = 0.020 |
| (x 10¹¹/g) | | | |
| S.E. | 0.16 | 0.25 | |

### Example 5; rumen simulation and field experiment

In this experiment, the effect of changing one fodder component in the diet used at milk farm 1 presented in Example 4 on the yield was studied via ruminal fermentation simulation and field testing.

The diet used on farm 1 was modified by replacing 1 kg of ELIT 2000 fodder with 1kg of Acetona fodder. Ruminal fermentation simulation was performed using the modified diet. With the change in the diet, the number of microbes increased by 17 %.

Table 5 presents the computational amounts of nutrients in the diets according to example 4 and in the modified diet according to the present example, based on a total quantity of 20 kg of dry matter.

**Table 5**

| | Farm 1 | Farm 2 | Farm 1 changed |
|---|---|---|---|
| Energy, MJ | 271 | 278 | 270 |
| AAT protein, g | 2260 | 2220 | 2200 |
| PBV protein, g | 150 | 570 | 90 |
| Raw protein., g | 3730 | 4200 | 3560 |
| Protein, % of | 18.65 | 21.00 | 17.80 |
| dry matter | | | |
| NDF fiber, g | 7990 | 6500 | 8050 |
| Starch, g | 1820 | 5400 | 1790 |
| Sugar, g | 370 | 1540 | 430 |
| E-fiber, g | 4780 | 4000 | 4790 |
| Readily soluble | 4920 | 3340 | 5090 |
| hh, g | | | |
| EFF protein, g | 2130 | 2750 | 2100 |

According to present-day knowledge, critical points in the animals' nutrient supply in the above-described diets are the supply of energy (MJ) and so-called bypass-protein (AAT), because, according to the standards, the above-mentioned quantities are sufficient for the production of 40 kg of energy-corrected milk (ECM), for which the standard need is 272 MJ of energy and 2060 g of AAT. The diet change does not seem to have any perceivable effect on the supply of energy and protein.

In the table, the PBV value describes a protein deficit/surplus, and its target value is near zero, although a small surplus is not detrimental. Surplus means a protein quantity that the microbes are unable to utilize and which then escapes from the rumen, ending up e.g. in urine.

The effects of two different diets on the yield were further studied via field testing on milk farm 1. The animals (18) in the test group were given a modified fodder mixture containing 1 kg of Acetona fodder according to this example, 2 weeks before and 2 months after calving. The control group consisted of animals (13) living on a diet as on milk farm 1.

The animals were observed for 7 months after calving via test milkings carried out once a month. The development of the yield was compared with the yield of corresponding test milkings of the same animals during the previous milking period (1 year), i.e. reference period. Table 6 shows the average milk yield (kg/d) and average protein yield (kg/d) of the animals in the control and test groups during the reference period and the milking period after calving.

**Table 6**

| | Control group | | Test group | |
|---|---|---|---|---|
| | Reference period | Testing period | Reference period | Testing period |
| Protein, g/day | 1026 | 1081 | 953 | 1099 |
| Milk yield, kg/day | 30.6 | 32.1 | 27.6 | 31.8 |

The results indicate that the animals in the control group increased their milk yield by 1.5 kg/day during 7 months as compared with the previous year. During the same period, the animals in the test group increased their milk yield by an average of 4.2 kg/day as compared with the previous year. The increase in net yield was 2.7 kg/day due to the diet change although the differences between the computational quantities of nutrients were small. Furthermore, the protein yield of the milk increased by an average of 55 g/day for the control group animals and by 146 g/day for the test group animals, i.e. the net increase was 91 g/day.

Based on the experiment, of the raw protein quantity eaten, about 29.0 % in the control group and about 31.0 % in the test group ended up in the milk protein of the animals. If the animals had been fed a fodder protein quantity as on farm 2 and if the yield had been as in the case of the farm 1 test group, then the nitrogen retention would have been 26.0 %. Correspondingly, the 91-g net protein yield for each animal corresponded to an increase of 14.72 g/day, i.e. during 7 months a total increase of 3.13 kg, of nitrogen fixation in microbial mass.

The effects of the two diets described above on the yield were also studied via a field experiment on milk farm 1 by using heifers calving for the first time as test animals. The animals (19) in the test group were given a modified fodder mixture according to this example for 2 weeks before and 2 months after calving. The control group consisted of animals (20) living on a diet according to milk farm 1.

The animals were observed for 7 months after calving via test milkings carried out once a month. Table 7 shows the daily average milk yield (kg) and average protein yield (kg) of the animals in the control and test groups during the milking period after calving.

**Table 7**

| | Control group | Test group |
|---|---|---|
| Protein, g/d | 841 | 959 |
| Milk yield, g/d | 24.3 | 28.1 |

The results indicate that the average milk yield of the animals in the test group was 3.8 kg/d and the average protein yield 118 g/d higher than in the control group. Based on the experiment, of the raw protein quantity eaten, about 26.5 % in the control group and about 31.7 % in the test group ended up in the milk protein of the animals. The amount of nitrogen contained in the urine of the test group animals was reduced by a total of about 8.9 g/animal during the test period.

Based on the experiments, the increased number of microbes in the rumen substantially increased the milk yield and the nitrogen fixation in the microbial mass.

### Example 6; rumen simulation

In this experiment, the effects of diet changes on fermentation products produced in the rumen were studied using ruminal fermentation simulation (example 1).

The modification of the diets by changing fodder components and their quantities is presented in Table 8 (the quantities of fodder components being given in terms of dry weight (kg)). The computational quantities of nutrients, based on a total amount of 20 kg of dry matter, are presented in Table 9. The simulation results for different diets are presented in Table 10.

Based on the microbial mass and the protein quantity in the diet, the nitrogen retention in the microbial mass, i.e. the N-fixation, was computed for different diets; the results are presented in Table 11.

As indicated by the results, Table 10, Acetona fodder increased microbial synthesis by 17 - 33 % without causing any substantial changes in gas production, total acids and acid relationships. Nitrogen fixation in the microbial mass increased by about 22 % when 1 kg of Elit 2000 fodder was replaced with Acetona fodder (1 kg), and by about 30 % when 1 kg of corn was replaced with Acetona fodder (1 kg). Based on the experiment, P-feed could be used to replace the pea without causing any substantial change in microbial synthesis. P-feed significantly reduced gas and methane production.

**Table 8**

| Feeding | 1 | 2 | 3 | 4 | 5 | 6 | 7 | 8 |
|---|---|---|---|---|---|---|---|---|
| Ensilage | 10 | 10 | 10 | 10 | 10 | 10 | 10 | 10 |
| Corn mixture | 2 | 2 | 2 | 4 | 4 | 6 | 6 | 5 |
| Elit 2000 | 6 | 5 | 6 | 4 | 4 | 2 | 2 | 2 |
| Acetona | | 1 | | | | | | 1 |
| Pea | 2 | 2 | | 2 | | 2 | | |
| P-feed | | | 2 | | 2 | | 2 | 2 |

**Table 9**

| Feeding | 1 | 2 | 3 | 4 | 5 | 6 | 7 | 8 |
|---|---|---|---|---|---|---|---|---|
| Dry matter ,kg | 20 | 20 | 20 | 20 | 20 | 20 | 20 | 20 |
| Energy, MJ | 249.2 | 248.4 | 240.0 | 247.0 | 237.8 | 244.8 | 235.6 | 235.9 |
| AAT prot., g | 2016 | 1950 | 1966 | 1868 | 1818 | 1720 | 1670 | 1678 |
| PBV prot., g | 538 | 463 | 754 | 440 | 656 | 342 | 558 | 532 |
| Raw prot., g | 3770 | 3610 | 3920 | 3472 | 3622 | 3174 | 3324 | 3313 |
| NDF fiber, g | 7230 | 7288 | 7030 | 7224 | 7024 | 7218 | 7018 | 7079 |
| E-fiber, g | 4880 | 4893 | 4880 | 4764 | 4764 | 4648 | 4648 | 4719 |
| ERP, g | 2038 | 2000 | 2198 | 1984 | 2144 | 1930 | 2090 | 2079 |
| Starch, g | 2528 | 2482 | 1428 | 3508 | 2408 | 4488 | 3388 | 2852 |
| Readily soluble hh, g | 4032 | 4215 | 4032 | 3528 | 3528 | 3024 | 3024 | 3459 |

**Table 10**

| Feeding | 1 | 2 | 3 | 4 | 5 | 6 | 7 | 8 |
|---|---|---|---|---|---|---|---|---|
| Gas production 12 h, ml | 120 | 119 | 110 | 131 | 124 | 126 | 121 | 116 |
| Total acids, mM | 92 | 94 | 90 | 101 | 97 | 95 | 91 | 95 |
| Number of microbes (x 10⁹) | 3.52 | 4.13 | 4.55 | 4.56 | 4.5 | 3.81 | 3.58 | 4.67 |
| Proportion of microbes, % | 100 | 117 | 129 | 129 | 128 | 108 | 102 | 133 |
| Acetic acid, % | 57 | 56 | 59 | 56 | 57 | 56 | 57 | 57 |
| Propionic acid, % | 27 | 28 | 26 | 29 | 28 | 28 | 27 | 27 |
| Butyric acid, % | 12.6 | 12.5 | 12.3 | 12.5 | 12.5 | 12.9 | 12.9 | 12.8 |
| Gas production 6 h, ml | 69 | 69 | 63 | 76 | 72 | 70 | 67 | 66 |
| Methane, 12 h, ml | 23.2 | 22.9 | 20.5 | 25.5 | 23.4 | 24.8 | 24.8 | 22.8 |

**Table 11**

| Feeding | 1 | 2 | 3 | 4 | 5 | 6 | 7 | 8 |
|---|---|---|---|---|---|---|---|---|
| N supply, g/kg DM | 30.16 | 28.88 | 31.36 | 27.78 | 28.98 | 25.39 | 26.50 | 26.59 |
| N in microbes, g/kg DM | 16.50 | 19.36 | 21.33 | 21.35 | 21.09 | 17.85 | 16.79 | 21.87 |
| Surplus N, g | 13.66 | 9.52 | 10.03 | 6.43 | 7.89 | 7.55 | 9.72 | 4.72 |
| N-fixation, % | 55 | 67 | 68 | 77 | 73 | 70 | 63 | 82 |

In summary, based on the above examples of its embodiments, we can state that the method of the invention is well applicable for the determination of the yield values of any diet for ruminants and for the testing and development of new as well as previously known fodder components. In particular, the invention makes it possible to evaluate the effects of different diet changes on the yield, which cannot be done on the basis of mere computational nutrient quantities. As indicated by the examples presented above, the microbes and fermentation products produced in ruminal fermentation simulation are proportional to the yield of the animal. It can be further seen from the embodiment examples that statistically significant differences regarding diet and yield are achieved by the method of the invention.

The embodiments of the invention are not restricted to the examples described above, but they can be varied in the scope of the following claims.

## Claims

1. Method for testing a fodder component for the feeding of ruminants, in which method one or more fodder components are tested via ruminal fermentation simulation in an artificial rumen to determine their properties so that the fodder component is decomposed by the action of microbes into fermentation products utilizable in the animal's metabolism and providing the nutritive substances needed by the animal, **characterized in that**, the fodder component is decomposed by the action of microbes of an inoculum comprising ruminal liquid which conditions and microbes correspond to the conditions and microbes in the animal's rumen, and fermentation products substantially proportional to the yield of the animal are measured, said fermentation products are compared with corresponding results achieved using known fodder components whose productional effect is known, and, based on the comparison, the effect of the fodder component on the animal's yield is determined.

2. Method as defined in claim 1, **characterized in that** ruminal fermentation simulation is carried out as a batch fermentation.

3. Method as defined in claim 1 or 2, **characterized in that** ruminal fermentation simulation is carried out in about 12 hours.

4. Method as defined in any of claims 1 - 3, **characterized in that** the effect of the fodder component on the yield is determined on the basis of one or more cycles of ruminal fermentation simulation.

5. Method as defined in any of claims 1 - 4, **characterized in that** the microbial mass produced in the simulation is measured.

6. Method as defined in any one of claims 1 - 5, **characterized in that** the number of microbes in a unit of weight and/or volume is measured.

7. Method as defined in any one of claims 1 - 6, **characterized in that** the fixation of nitrogen in the microbial mass is measured.

8. Method as defined in any one of claims 1 - 7, **characterized in that** the total gas production, total acids, acetic acid, propionic acid, butyric acid and/or the overall profile of the products are/is measured.

9. Method as defined in any one of claims 1 - 7, **characterized in that** the fermentation products are measured qualitatively.

10. Method as defined in any one of claims 1 - 9, **characterized in that** the fermentation products are measured quantitatively.

11. Method as defined in any one of claims 1 - 10, **characterized in that** the fodder component tested via ruminal fermentation simulation is tested via a field experiment in which the animals are divided into two groups, a control group and a test group, which are fed according to a substantially similar basic diet; the animals in the test group are fed the fodder component to be tested during an exactly defined period of time; and the yield of the animals of the control and test groups is measured and the effect of the fodder component on the yield is measured by comparing the yields of the two groups with each other.

12. Method as defined in any one of claims 1 - 11, **characterized in that**, in the field experiment, the effect of the fodder component on the yield is determined by comparing the measured yield of the animals of the test and control groups to a reference level, which is defined by measuring the yield of each animal and/or group from a given earlier period during which each animal is fed according to a substantially similar diet.

13. Method as defined in any one of claims 1 - 12, **characterized in that** the reference level is defined by measuring the yield of the animal during one year.

14. Method as defined in any one of claims 1 - 13, **characterized in that** the effect of the fodder component on the yield before and/or after calving is determined via a field experiment.

15. Method as defined in any one of claims 1 - 14, **characterized in that** statistically significant differences regarding the fodder component and yield are determined via a field experiment.

16. Method as defined in any one of claims 1 - 15, **characterized in that**, in the field experiment, the milk production of cows is tested and a test milking is performed at exactly defined intervals to measure the yield.

## Patentansprüche

1. Verfahren zum Testen eines Futterbestandteils zum Füttern von Wiederkäuern, wobei bei dem Verfahren ein oder mehrere Futterbestandteile über Simulation ruminaler Fermentation in einem künstlichen Rumen getestet werden, um ihre Eigenschaften zu bestimmen, sodass der Futterbestandteil durch die Wirkung von Mikroben in Fermentationsprodukte zersetzt wird, die im Stoffwechsel des Tiers genutzt werden können und von dem Tier benötigte Nährstoffe bereitstellen, **dadurch gekennzeichnet, dass** der Futterbestandteil durch die Wirkung von Mikroben eines Inokulums zersetzt wird, das ruminale Flüssigkeit umfasst und dessen Bedingungen und Mikroben den Bedingungen und Mikroben im Rumen des Tieres entsprechen, und Fermentationsprodukte im Wesentlichen proportional zur Leistung des Tieres gemessen werden, wobei die Fermentationsprodukte mit entsprechenden Ergebnissen verglichen werden, die bei der Verwendung bekannter Futterbestandteile erzielt werden, deren Produktionseffekt bekannt ist, und auf Basis des Vergleichs der Effekt des Futterbestandteils auf die Leistung des Tiers bestimmt wird.

2. Verfahren nach Anspruch 1, **dadurch gekennzeichnet, dass** die Simulation ruminaler Fermentation als eine chargenweise Fermentation ausgeführt wird.

3. Verfahren nach Anspruch 1 oder 2, **dadurch gekennzeichnet, dass** die ruminale Fermentation in ungefähr 12 Stunden ausgeführt wird.

4. Verfahren nach einem der Ansprüche 1 - 3, **dadurch gekennzeichnet, dass** der Effekt des Futterbestandteils auf die Leistung auf Basis eines oder mehrer Zyklen von Simulation ruminaler Fermentation bestimmt wird.

5. Verfahren nach einem der Ansprüche 1 - 4, **dadurch gekennzeichnet, dass** die mikrobielle Masse, die bei der Simulation erzeugt wird, gemessen wird.

6. Verfahren nach einem der Ansprüche 1 - 5, **dadurch gekennzeichnet, dass** die Zahl von Mikroben in einer Gewichts- und/oder Volumeneinheit gemessen wird.

7. Verfahren nach einem der Ansprüche 1-6, **dadurch gekennzeichnet, dass** die Fixierung von Stickstoff in der mikrobiellen Masse gemessen wird.

8. Verfahren nach einem der Ansprüche 1 - 7, **dadurch gekennzeichnet, dass** die gesamte Gaserzeugung, die gesamte Säuremenge, Essigsäure, Propionsäure, Buttersäure und/oder das gesamte Profil der Produkte gemessen wird/werden.

9. Verfahren nach einem der Ansprüche 1 - 7, **dadurch gekennzeichnet, dass** die Fermentationsprodukte qualitativ gemessen werden.

10. Verfahren nach einem der Ansprüche 1 - 9, **dadurch gekennzeichnet, dass** die Fermentationsprodukte quantitativ gemessen werden.

11. Verfahren nach einem der Ansprüche 1 - 10, **dadurch gekennzeichnet, dass** der Futterbestandteil, der über Simulation ruminaler Fermentation getestet wird, über einen Feldversuch getestet wird, in dem die Tiere in zwei Gruppen unterteilt werden, das heißt eine Kontrollgruppe und eine Testgruppe, die gemäß einer im Wesentlichen gleichen Grundernährung gefüttert werden, wobei die Tiere in der Testgruppe während eines genau definierten Zeitraums mit dem zu testenden Futterbestandteil gefüttert werden und die Leistung der Tiere der Kontroll- und der Testgruppe gemessen werden und die Auswirkung des Futterbestandteils auf die Leistung durch Vergleichen der Leistung der zwei Gruppen miteinander gemessen wird.

12. Verfahren nach einem der Ansprüche 1 - 11, **dadurch gekennzeichnet, dass** in dem Feldversuch der Effekt des Futterbestandteils auf die Leistung bestimmt wird, indem die gemessene Leistung der Tiere der Test- und der Kontrollgruppen mit einem Bezugspegel verglichen wird, der durch Messen der Leistung jedes Tiers und/oder jeder Gruppe gegenüber einer bestimmten früheren Periode definiert wird, während der jedes Tier gemäß einer im Wesentlichen gleichen Ernährung gefüttert wird.

13. Verfahren nach einem der Ansprüche 1 - 12, **dadurch gekennzeichnet, dass** der Bezugspegel definiert wird, indem die Leistung des Tieres während eines Jahres gemessen wird.

14. Verfahren nach einem der Ansprüche 1 -13, **dadurch gekennzeichnet, dass** der Effekt des Futterbestandteils auf den Ertrag vor und/oder nach dem Kalben über einen Feldversuch bestimmt wird.

15. Verfahren nach einem der Ansprüche 1 -14, **dadurch gekennzeichnet, dass** statistisch signifikante Differenzen bezüglich des Futterbestandteils und der Leistung über einen Feldversuch bestimmt werden.

16. Verfahren nach einem der Ansprüche 1 - 15, **dadurch gekennzeichnet, dass** in dem Feldversuch die Milchproduktion von Kühen getestet wird und ein Testmelken in genau definierten Intervallen durchgeführt wird, um die Leistung zu messen.

## Revendications

1. Procédé pour tester un produit de fourrage destiné à nourrir des ruminants, dans lequel un ou plusieurs produits de fourrage sont testés par l'intermédiaire d'une simulation de fermentation ruminale dans une panse artificielle afin de déterminer leurs propriétés de sorte que le produit de fourrage soit décomposé par l'action de microbes en des produits de fermentation utilisables dans le métabolisme de l'animal et fournissant les substances nutritives dont l'animal a besoin, **caractérisé en ce que** le produit de fourrage est décomposé par l'action de microbes dans un inoculum comprenant un liquide ruminal dont les conditions et les microbes correspondent aux conditions et aux microbes présents dans la panse de l'animal, et les produits de la fermentation sensiblement proportionnels au rendement de l'animal sont mesurés, lesdits produits de fermentation sont comparés avec les résultats correspondants obtenus en utilisant des produits de fourrage connus dont l'effet productif est connu, et, sur la base de la comparaison, l'effet du produit de fourrage sur le rendement de l'animal est déterminé.

2. Procédé selon la revendication 1, **caractérisé en ce que** la simulation de la fermentation ruminale est réalisée sous forme d'une fermentation par lots.

3. Procédé selon la revendication 1 ou 2, **caractérisé en ce que** la simulation de la fermentation ruminale est réalisée en environ 12 heures.

4. Procédé selon l'une quelconque des revendications 1 à 3, **caractérisé en ce que** l'effet du produit de fourrage sur le rendement est déterminé sur la base d'un ou de plusieurs cycles de simulation de la fermentation ruminale.

5. Procédé selon l'une quelconque des revendications 1 à 4, **caractérisé en ce que** la masse microbienne produite dans la simulation est mesurée.

6. Procédé selon l'une quelconque des revendications 1 à 5, **caractérisé en ce que** le nombre de microbes dans une unité de poids et/ou de volume est mesuré.

7. Procédé selon l'une quelconque des revendications 1 à 6, **caractérisé en ce que** la fixation de l'azote dans la masse microbienne est mesurée.

8. Procédé selon l'une quelconque des revendications 1 à 7, **caractérisé en ce que** la production gazeuse totale, les acides totaux, l'acide acétique, l'acide propionique, l'acide butyrique et/ou le profil global des produits sont/est mesuré(s).

9. Procédé selon l'une quelconque des revendications 1 à 7, **caractérisé en ce que** les produits de fermentation sont mesurés qualitativement.

10. Procédé selon l'une quelconque des revendications 1 à 9, **caractérisé en ce que** les produits de fermentation sont mesurés quantitativement.

11. Procédé selon l'une quelconque des revendications 1 à 10, **caractérisé en ce que** le produit de fourrage testé par l'intermédiaire d'une simulation de la fermentation ruminale est testé par l'intermédiaire d'une expérience en champ dans laquelle les animaux sont divisés en deux groupes, un groupe témoin et un groupe de test, qui sont nourris selon un régime de base sensiblement similaire ; les animaux du groupe témoin sont nourris avec le produit de fourrage devant être testé pendant une période de temps exactement définie ; et le rendement des animaux des groupes témoin et de test est mesuré et l'effet du produit de fourrage sur le rendement est mesuré en comparant les rendements des deux groupes entre eux.

12. Procédé selon l'une quelconque des revendications 1 à 11, **caractérisé en ce que**, dans l'expérience en champ, l'effet du produit de fourrage sur le rendement est déterminé en comparant le rendement mesuré des animaux des groupes de test et témoin avec un niveau de référence, qui est défini en mesurant le rendement de chaque animal et/ou groupe à partir d'une période antérieure donnée lors de laquelle chaque animal est nourri selon un régime sensiblement similaire.

13. Procédé selon l'une quelconque des revendications 1 à 12, **caractérisé en ce que** le niveau de référence est défini en mesurant le rendement de l'animal pendant un an.

14. Procédé selon l'une quelconque des revendications 1 à 13, **caractérisé en ce que** l'effet du produit de fourrage sur le rendement avant et/ou après le vêlage est déterminé par l'intermédiaire d'une expérience en champ.

15. Procédé selon l'une quelconque des revendications 1 à 14, **caractérisé en ce que** des différences statistiquement significatives concernant le produit de fourrage et le rendement sont déterminées par l'intermédiaire d'une expérience en champ.

16. Procédé selon l'une quelconque des revendications 1 à 15, **caractérisé en ce que**, dans l'expérience en champ, la production de lait des vaches est testée et une traite de test est réalisée à des intervalles exactement définis afin de mesurer le rendement.
